# EUROPEAN PATENT APPLICATION

(11) **EP 2 695 580 A1**
(43) Date of publication of application: **12.02.2014**
(21) Application number: 13160388.8
(22) Date of filing: 21.03.2013
(51) Int. Cl.: A61B 6/03, A61B 6/00

(54) **Radiographic image photographing method and apparatus**

(30) Priority: 06.08.2012 KR 20120086004
(71) Applicant: Samsung Electronics Co., Ltd, Gyeonggi-do 443-742 (KR)
(72) Inventor: Park, Jun-young, 384-38 Gyeonggi-do (KR)
(74) Representative: Land, Addick Adrianus Gosling

(57) **Abstract**

A radiographic image photographing method using a radiographic image photographing apparatus. The radiation image photographing method includes: displaying a parameter value of the radiographic image photographing apparatus for a predetermined part of a target object and a preview image corresponding to the parameter value; changing the parameter value or the preview image based on a user's input; and changing the preview image according to the changed parameter value, or the parameter value according to the changed preview image.

## Description

This application claims the benefit of Korean Patent Application No. 10-2012-0086004, filed on August 6, 2012, in the Korean Intellectual Property Office, the disclosure of which is incorporated herein in its entirety by reference.

The present invention relates to a radiographic image photographing method and apparatus, and more particularly, to a radiographic image photographing method and apparatus that simultaneously display a parameter value of the radiographic image photographing apparatus and a preview image.

Radiographic image photographing apparatuses are used to observe a structure of the inside of an organic body by using radioactive rays. The radiographic image photographing apparatuses include a computed tomography (CT) photographing apparatus, a positron emission tomography (PET) photographing apparatus, etc.

The CT image photographing apparatus captures a CT image by using a prototype machine including an X-ray generation apparatus and acquires a cross-sectional image obtained horizontally across a human body, unlike simple X-ray photographing. A small part of a structure overlaps in the CT image, compared to the simple X-ray photographing, and thus, the structure and a lesion can be more clearly observed.

The PET photographing apparatus is one of nuclear medicine testing apparatuses capable of 3-dimensionally representing a physiological, chemical, and functional image of a human body by using radiation medicines that emit positrons.

Radiographic image photographing apparatuses emit radioactive rays onto the human body, which causes exposure of the human body to radiation. Although the human body is exposed to radiation of about 2 ∼ 10 mSv according to a radiographic image photographing method, this value corresponds to an amount of radiation of daily exposure for about 8 months to about 3 years. However, radiation by the radiographic image photographing apparatuses is likely to cause a serious disease and develop complications for a fetus of a pregnant woman, and adversely affect the growth of the fetus. Accordingly, there is a need to reduce exposure of radiation by the radiographic image photographing apparatus.

Additional aspects and/or advantages will be set forth in part in the description which follows and, in part, will be apparent from the description, or may be learned by practice of the invention.

The present invention provides a radiographic image photographing apparatus and method capable of simultaneously displaying a parameter value of the radiographic image photographing apparatus and a preview image corresponding to the parameter value, thereby efficiently reducing an amount of radiation to which a human body is exposed.

According to an aspect of the present invention, there is provided a radiographic image photographing method using a radiographic image photographing apparatus, the radiographic image photographing method including: displaying a parameter value of the radiographic image photographing apparatus for a predetermined part of a target object and a preview image corresponding to the parameter value; changing the parameter value or the preview image based on a user's input; and changing the preview image according to the changed parameter value, or the parameter value according to the changed preview image.

The displaying of the preview image may include: displaying the preview image corresponding to the predetermined part of the target object.

The displaying of the preview image may include: displaying the preview image corresponding to at least one of a tube voltage, a tube current, a scan type, the number of images, spaces between images, start and end points of each image, a scan field of view (SFOV), a thickness of each image, and a reconfiguration algorithm included in the parameter value.

The changing of the preview image according to the changed parameter value may include: changing at least one of spatial resolution, contrast resolution, and an amount of noise of the preview image according to a change level of at least one of the tube current and the tube voltage included in the parameter value.

The changing of the preview image according to the changed parameter value may include: changing at least one of the tube current and the tube voltage included in the parameter value according to a change level of at least one of spatial resolution, contrast resolution, and an amount of noise of the preview image.

The displaying may include: displaying the parameter value on a first region of a display unit of the radiographic image photographing apparatus, and the preview image on a second region thereof.

The displaying of the preview image may include: displaying a preview image corresponding to examinee information.

The examinee information may include at least one of age, weight, height, a body mass index (BMI), and gender of an examinee.

The radiographic image photographing method may further include: obtaining a radiographic image of the predetermined part of the target object according to the changed parameter value; changing at least one of spatial resolution, contrast resolution, and an amount of noise of the preview image based on at least one of spatial resolution, contrast resolution, and an amount of noise of the obtained radiographic image; and storing the preview image having the changed at least one of the spatial resolution, the contrast resolution, and the amount of noise as an updated preview image.

The radiographic image photographing method may further include: obtaining a radiographic image of the predetermined part of the target object according to the changed parameter value; receiving evaluation information regarding the preview image from a user; changing at least one of spatial resolution, contrast resolution, and an amount of noise of the preview image according to the received evaluation information; and storing the preview image having the changed at least one of the spatial resolution, the contrast resolution, and the amount of noise as an updated preview image.

The preview image may include a graph image indicating an amount of radiation with respect to the parameter value.

According to another aspect of the present invention, there is provided an apparatus for photographing a radiographic image of a target object, the apparatus including: a display unit for displaying a parameter value of the radiographic image photographing apparatus for a predetermined part of the target object and a preview image corresponding to the parameter value; and a control unit for changing the parameter value or the preview image based on a user's input, wherein the control unit changes the preview image according to the changed parameter value, or the parameter value according to the changed preview image.

The display unit may display the preview image corresponding to the predetermined part of the target object.

The display unit may display the preview image corresponding to at least one of a tube voltage, a tube current, a scan type, the number of images, spaces between images, start and end points of each image, a scan field of view (SFOV), a thickness of each image, and a reconfiguration algorithm included in the parameter value.

The control unit may change at least one of spatial resolution, contrast resolution, and an amount of noise of the preview image according to a change level of at least one of the tube current and the tube voltage included in the parameter value.

The control unit may change at least one of the tube current and the tube voltage included in the parameter value according to a change level of at least one of spatial resolution, contrast resolution, and an amount of noise of the preview image.

The display unit may display the parameter value on a first region thereof, and the preview image on a second region thereof.

The display unit may display a preview image corresponding to examinee information.

The examinee information may include at least one of age, weight, height, a body mass index (BMI), and gender of an examinee.

The apparatus may further include: an image obtaining unit for obtaining a radiographic image of the predetermined part of the target object according to the changed parameter value, wherein the control unit changes at least one of spatial resolution, contrast resolution, and an amount of noise of the preview image based on at least one of spatial resolution, contrast resolution, and an amount of noise of the obtained radiographic image, and stores the preview image having the changed at least one of the spatial resolution, the contrast resolution, and the amount of noise as an updated preview image.

The apparatus may further include: an image obtaining unit for obtaining a radiographic image of the predetermined part of the target object according to the changed parameter value, wherein the control unit changes at least one of spatial resolution, contrast resolution, and an amount of noise of the preview image according to evaluation information received from the user, and stores the preview image having the changed at least one of the spatial resolution, the contrast resolution, and the amount of noise as an updated preview image.

The preview image may include a graph image indicating an amount of radiation with respect to the parameter value.

According to another aspect of the present invention, there is provided a computer readable recording medium having recorded thereon a program for executing the radiographic image photographing method.

### BRIEF DESCRIPTION OF THE DRAWINGS

The above and other features and advantages of the present invention will become more apparent by describing in detail exemplary embodiments thereof with reference to the attached drawings in which:
FIG. 1 is a block diagram of a display unit for setting a parameter value of a conventional radiographic image photographing apparatus;
FIG. 2A is a block diagram of a radiographic image photographing apparatus according to an embodiment of the present invention, and FIG. 2B is a block diagram of a radiographic image photographing apparatus according to another embodiment of the present invention;
FIG. 3 is a block diagram of a display unit for setting a parameter value of a radiographic image photographing apparatus, according to an embodiment of the present invention;
FIG. 4A and 4B are radiographic images captured by using a radiographic image photographing apparatus according to an embodiment of the present invention;
FIG. 5 is a flowchart of a radiographic image photographing method according to an embodiment of the present invention;
FIG. 6 is a flowchart of a preview image updating method in a radiographic image photographing method, according to an embodiment of the present invention; and
FIG. 7 is a flowchart of a preview image updating method in a radiographic image photographing method, according to another embodiment of the present invention.

### DETAILED DESCRIPTION

Merits and features of the present invention, and a method for accomplishing the merits and features will now be described more fully with reference to the accompanying drawings, in which exemplary embodiments of the invention are shown. The invention may, however, be embodied in many different forms and should not be construed as being limited to the embodiments set forth herein; rather, these embodiments are provided so that this disclosure will be thorough and complete, and will fully convey the concept of the invention to those of ordinary skill in the art. The scope of the invention is defined not by the detailed description of the invention but by the appended claims. Like reference numerals in the drawings denote like elements.

The terms "unit" as used herein may refer to, but is not limited to, a software or hardware component, such as a Field Programmable Gate Array (FPGA) or Application Specific Integrated Circuit (ASIC), which performs certain tasks. A unit may be configured to reside on an addressable storage medium and configured to be executed on one or more processors. Thus, examples of a unit may include components, such as software components, object-oriented software components, class components and task components, processes, functions, attributes, procedures, subroutines, segments of program code, drivers, firmware, microcode, circuitry, data, database, data structures, tables, arrays, and variables. The functionality provided in the components and units may be combined into fewer components and units or further separated into additional components and units.

Expressions such as at least one of," when preceding a list of elements, modify the entire list of elements and do not modify the individual elements of the list.

A "target object" used herein includes a human or animal whose radiographic image is captured.

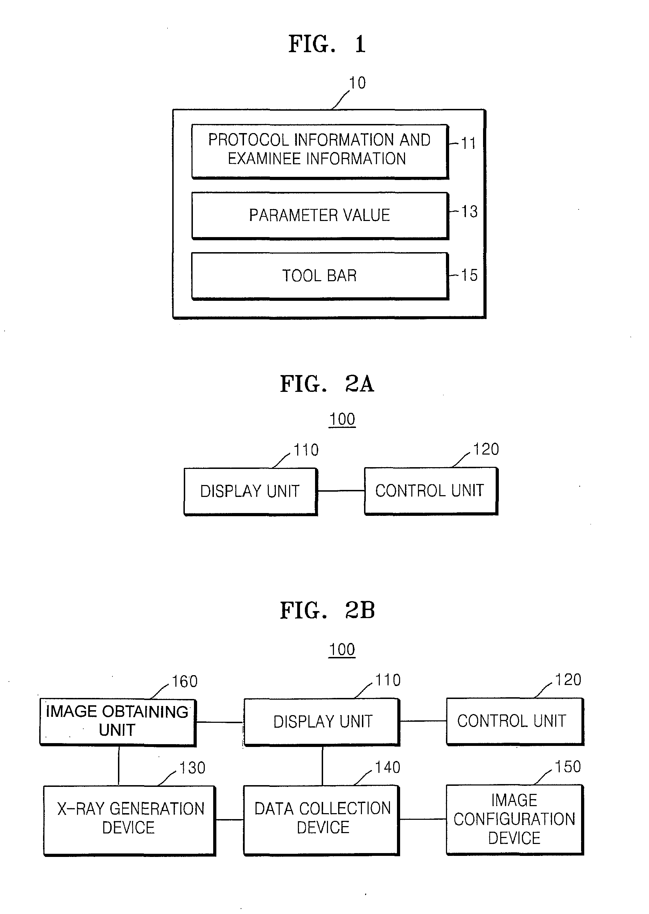

FIG. 1 is a block diagram of a display unit 10 for setting a parameter value of a conventional radiographic image photographing apparatus.

Referring to FIG. 1, the display unit 10 for setting the parameter value of the conventional radiographic image photographing apparatus may display protocol information and examinee information 11, a parameter value 13, or a tool bar 15.

The protocol information includes information regarding a predetermined part of a target object whose radiographic image is captured. For example, the protocol information may include information regarding each part of the inside of a human body such as a head, body, abdomen, and breast of a human.

The examinee information includes information regarding the target object for whom radiographic images are captured, and may include at least one of the age, weight, height, body mass index (BMI), and sex of an examinee. In this regard, the BMI means an amount of fat estimated from the height and weight of the examinee.

The parameter value 13 is a set value of the conventional radiographic image photographing apparatus to manipulate the conventional radiographic image photographing apparatus and may be differently set for each part of the target object.

The parameter value 13 may include at least one of a scan type indicating a radiographic image photographing method, the number of images obtained by the conventional radiographic image photographing apparatus, spaces between images, a start point of the target object indicating a point of starting radiographic image photographing, an end point of the target object indicating a point of ending radiographic image photographing, an image thickness indicating a thickness of a cross-section of a radiographic image, a scan field of view (SFOV) indicating an interest region used to obtain data during scanning, a reconfiguration algorithm indicating a reconfiguration method of a computed tomography (CT) image, a tube voltage that is an electric potential applied between a negative electrode and a positive electrode of an X-ray tube, and a tube current that is a positive electrode current flowing in an electronic beam colliding with the positive electrode of the X-ray tube during the X-ray radiation. The scan type may include an axial scan, a spiral scan, or a cine scan.

The axial scan is a method of capturing a radiographic image by using the X-ray tube rotating around an examinee still on an examining table and then capturing the radiographic image after moving the examining table. The spiral scan is a method of capturing a radiographic image through a high speed continuous spiral scan by using a slip ring technology instead of a high voltage cable. The cine scan is a method of continuously capturing a single cross-section of a radiographic image at the same location of the target object at a predetermined time interval.

The reconfiguration algorithm may include a filtered back projection, an iterative reconstruction, etc. In general, a radiographic image reconfigured by using the iterative reconstruction has a better resolution than a radiographic image reconfigured by using the filtered back projection.

The parameter value 13 may be previously set by a manufacturing company of the conventional radiographic image photographing apparatus for each part of the target object and may be optionally set by a user.

The tool bar 15 is an interface for changing the protocol information or the parameter value 13.

Conventionally, a user optionally sets the parameter value 13 for each part of the target object based on experience of the user or uses the parameter value 13 set by the manufacturing company. The tube voltage and the tube current included in the parameter value 13 are closely related to an amount of radiation. The user tends to set a high tube voltage and a high tube current for a radiographic image with better quality.

Conventionally, the user sets a high tube voltage and a high tube current in order to prevent the radiographic image from being captured more than twice. However, this problematically increases an amount of radiation exposure to the examinee. An embodiment of the present invention simultaneously displays a parameter value and a preview image corresponding to the parameter value to the user so that the user may efficiently adjust an amount of radiation.

FIG. 2A is a block diagram of a radiographic image photographing apparatus 100 according to an embodiment of the present invention.

Referring to FIG. 2A, the radiographic image photographing apparatus 100 according to an embodiment of the present invention may include a display unit 110 and a control unit 120.

The display unit 110 displays a parameter value of the radiographic image photographing apparatus 100 with respect to a predetermined part of a target object and a preview image corresponding to the parameter value. The preview image may be identified according to the parameter value for each part of the target object and stored in a memory unit (not shown) of the radiographic image photographing apparatus 100.

As described above, the parameter value of the radiographic image photographing apparatus 100 may include a tube voltage, a tube current, a scan type, the number of images, spaces between images, start and end points of each image, an SFOV, a thickness of each image, and a reconfiguration algorithm. The display unit 110 may display preview images corresponding to the above parameter values.

For example, if a user selects the abdomen of the target object as a part for which a radiographic image is to be captured, the display unit 110 may display a parameter value of the radiographic image photographing apparatus 100 set for the abdomen and a preview image of the abdomen that is expected to be obtained according to the parameter value.

If the preview image of the abdomen is not stored in the memory unit of the radiographic image photographing apparatus 100, the display unit 110 may display a preview image corresponding to a part of the parameter values of the radiographic image photographing apparatus 100. That is, the display unit 110 may display a preview image of the head corresponding to at least one of the tube voltage and the tube current included in the parameter values.

The preview image displayed by the display unit 110 may include a graph image indicating an amount of radiation according to the parameter value. More specifically, the display unit 110 may display an amount of radiation of the tube voltage and the tube current in the graph image so that the user may easily know the amount of radiation of the tube voltage and the tube current.

Also, the display unit 110 may display preview images corresponding to parameter values and examinee information. More specifically, the display unit 110 may display a preview image corresponding to at least one of examinee's age, height, weight, BMI, and gender among preview images corresponding to parameter values. For example, in the case of a child examinee, the display unit 110 may display a preview image in consideration of the child's age, height, weight, BMI, and gender, and in the case of an adult examinee, the display unit 110 may display a preview image in consideration of the adult's age, height, weight, BMI, and gender. In this case, preview images may be identified according to the parameter value for each part of the target object and the examinee information and stored in the memory unit. Accordingly, the display unit 110 may provide the user with more reliable preview images.

The control unit 120 changes the parameter values or the preview images based on a user's input. Although not shown in FIG. 2A, the control unit 120 may be connected to an input unit such as a keyboard, a voice command, a mouse, and a touch panel. by which the user may control the radiographic image photographing apparatus 100. The control unit 120 receives the user's input through the input unit.

The user may select one of the parameter values by using the keyboard, the voice command, or the mouse and change the selected parameter value. Also, the user may change at least one of resolution and an amount of noise of the preview image displayed on the display unit 110, by using the keyboard, the voice command, or the mouse.

The control unit 120 changes the preview image displayed on the display unit 110 according to the parameter value changed based on the user's input. Alternatively, the control unit 120 may change the parameter value according to the preview image changed based on the user's input. That is, the parameter value and the preview image may be interconnected with each other and changed.

More specifically, the control unit 120 may change at least one of spatial resolution, contrast resolution, and an amount of noise of the preview image according to a change level of at least one of the tube current and the tube voltage included in the parameter values. Also, the control unit 120 may change at least one of the spatial resolution, the contrast resolution, and the amount of noise of the preview image according to the change level of at least one of the parameter values including the scan type, the number of images, spaces between images, start and end points of each image, the SFOV, a thickness of each image, and the reconfiguration algorithm.

For example, if the user changes the tube current or the tube voltage, a preview image having reduced resolution or an increased amount of noise may be displayed. Also, if the user changes the scan type from an axial scan to a spiral scan, a preview image including spiral artifact may be displayed.

The spatial resolution means a space or angle between two objects that may be identified by using the radiographic image photographing apparatus 100. The contrast resolution means a degree used to identify a difference such as brightness of an image.

The control unit 120 may change at least one of the tube current and the tube voltage included in the parameter values according to the change level of at least one of the spatial resolution, the contrast resolution, and the amount of noise of the preview image. That is, the control unit 120 may change at least one of the tube voltage and the tube current so as to obtain a radiographic image identical or similar to the preview image changed by the user. Also, the control unit 120 may change at least one of the parameter values including the scan type, the number of images, spaces between images, start and end points of each image, the SFOV, a thickness of each image, and the reconfiguration algorithm other than the tube voltage and the tube current according to the change level of at least one of the spatial resolution, the contrast resolution, and the amount of noise of the preview image.

The user may acknowledge a radiographic image that is to be substantially obtained through the preview image displayed on the display unit 110 and change a parameter value in such a way that the user may identify a preview image, thereby preventing the tube voltage or the tube current from having a high value. Accordingly, an amount of radiation exposure to the examinee may be reduced.

As shown in FIG. 2B, the radiographic image photographing apparatus 100 according to an embodiment of the present invention may further include an X-ray generation device 130, a data collection device 140, or an image configuration device 150. The X-ray generation device 130, the data collection device 140, or the image configuration device 150 may be elements for obtaining a radiographic image according to a parameter value set by a user.

The X-ray generation device 130 is for accelerating thermally emitting electrons of a filament in a vacuum tube at a high voltage between metals such as tungsten, molybdenum, and copper and colliding the accelerated electrons with a metal target, thereby generating X-rays.

The data collection device 140 is for collecting predetermined data from the target object by using the X-rays, converting the collected data into an electrical signal, and converting the electrical signal into a digital image signal.

The image configuration device 150 is for reconfiguring the data obtained from the data collection device 140, and performs pre-processing, convolution, and back-projection operations. The pre-processing, convolution, and back-projection operations are obvious to one of ordinary skill in the radiology field, and thus detailed descriptions thereof are omitted here.

A tool bar 115 is an interface for changing a protocol information or a parameter value 113.

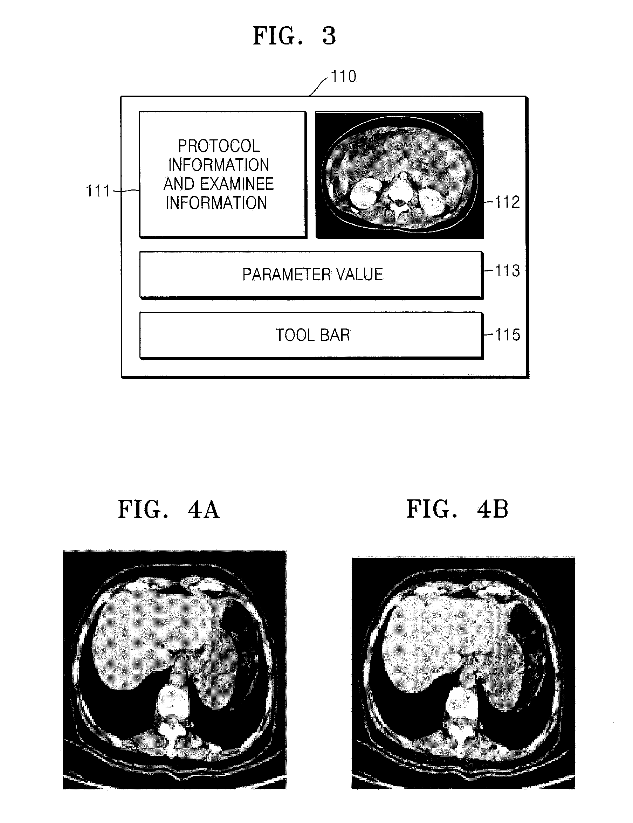

FIG. 3 is a block diagram of the display unit 110 for setting a parameter value 113 of the radiographic image photographing apparatus 100, according to an embodiment of the present invention. The display unit 110 may display the parameter value 113 on a first region of the display unit 110, and a preview image 112 on a second region of the display unit 110.

The radiographic image photographing apparatus 100 according to another embodiment of the present invention may continuously update a preview image stored in a memory unit and reliably provide the preview image.

More specifically, the radiographic image photographing apparatus 100 according to another embodiment of the present invention may further include an image obtaining unit 160. The image obtaining unit 160 obtains a radiographic image of a predetermined part of a target object according to a parameter value changed by a user.

The control unit 120 may change at least one of a spatial resolution, a contrast resolution, and an amount of noise of the preview image stored in the memory unit based on at least one of the spatial resolution, contrast resolution, and an amount of noise of the obtained radiographic image, and store the preview image having the changed at least one of the spatial resolution, the contrast resolution, and the amount of noise in the memory unit as the updated preview image.

For example, in a case where the user sets a parameter value based on a preview image displayed on the display unit 110 and captures the radiographic image with respect to the predetermined part of the target object according to the set parameter value, the actually captured radiographic image and the preview image may differ from each other. In this case, the control unit 120 changes at least one of the spatial resolution, the contrast resolution, and the amount of noise of the preview image according to the spatial resolution, the contrast resolution, and the amount of noise of the radiographic image to increase the similarity between the actually captured radiographic image and the preview image.

Also, the control unit 120 may receive evaluation information regarding the preview image from the user, and change at least one of the spatial resolution, the contrast resolution, and the amount of noise of the preview image according to the received evaluation information. The control unit 120 may store the preview image having the changed at least one of the spatial resolution, the contrast resolution, and the amount of noise in the memory unit as the updated preview image.

For example, the user may evaluate a similarity between the radiographic image and the preview image with respect to the radiographic image obtained by the image obtaining unit 160. In this case, the control unit 120 may change at least one of the spatial resolution, the contrast resolution, and the amount of noise of the preview image according to the evaluation information to increase the similarity between the actually captured radiographic image and the preview image based on the evaluation information received from the user.

FIG. 4A and 4B are radiographic images captured by using a radiographic image photographing apparatus according to an embodiment of the present invention.

FIG. 4A shows the radiographic image captured by a user at a tube current of 640 mA. FIG. 4B shows the radiographic image captured by the user at a tube current of 20 mA according to an aspect of the embodiment. Although the radiographic image of FIG. 4A has a greater amount of noise than that of FIG. 4B, such a difference may be identified by the user. The tube current is set low, which may reduce an amount of radiation exposure to the user.

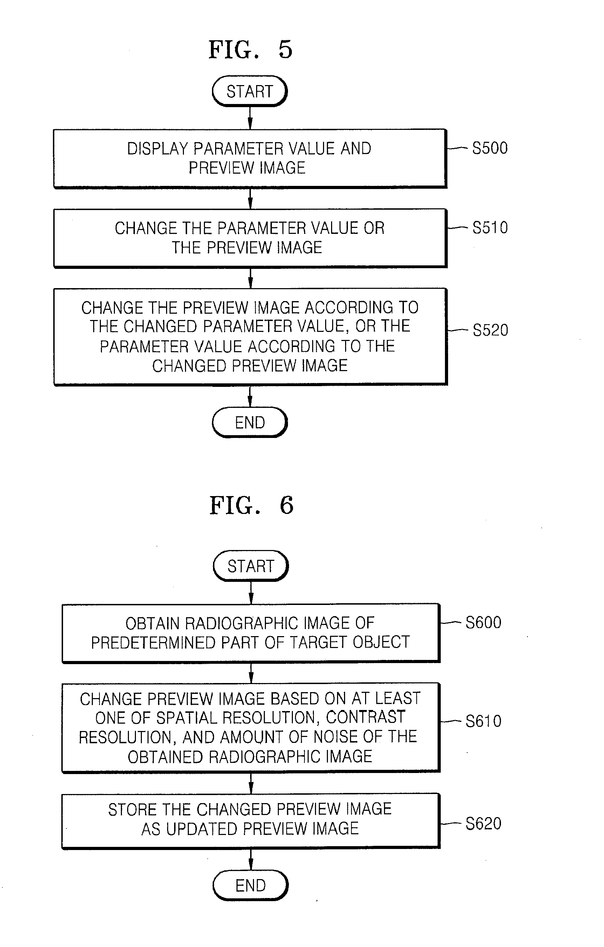

FIG. 5 is a flowchart of a radiographic image photographing method according to an embodiment of the present invention. Referring to FIG. 5, the radiographic image photographing method according to an embodiment of the present invention includes operations sequentially performed by the radiographic image photographing apparatus 100 of FIG. 2. Accordingly, the description given above with respect to the radiographic image photographing apparatus 100 of FIG. 2 may also be applied to the radiographic image photographing method of FIG. 5.

In operation S500, the radiographic image photographing apparatus 100 displays a parameter value of the radiographic image photographing apparatus 100 for a predetermined part of a target object and a preview image corresponding to the parameter value. The radiographic image photographing apparatus 100 may display the parameter value on a first region of the display unit 110 and the preview image on a second region of the display unit 110.

In operation S510, the radiographic image photographing apparatus 100 changes the parameter value or the preview image based on a user's input. The user may change the parameter value or the preview image by using a keyboard, a voice command, a mouse, a touch panel, etc. However, it is not limited thereto.

In operation S520, the radiographic image photographing apparatus 100 may change the preview image according to the changed parameter value, or the parameter value according to the changed preview image. More specifically, the radiographic image photographing apparatus 100 may change at least one of an amount of noise, a spatial resolution, and a contrast resolution of the preview image according to a change level of at least one of a tube current and a tube voltage included in the parameter value or change at least one of the tube current and the tube voltage included in the parameter value according to a change level of at least one of the amount of noise, the spatial resolution, and the contrast resolution of the preview image.

The user may reduce an amount of radiation by adjusting the parameter value and the preview image corresponding to the parameter value.

FIG. 6 is a flowchart of a preview image updating method in a radiographic image photographing method according to an embodiment of the present invention.

In operation S600, the radiographic image photographing apparatus 100 obtains a radiographic image of a predetermined part of a target object according to a parameter value set by a user.

In operation S610, the radiographic image photographing apparatus 100 changes at least one of spatial resolution, contrast resolution, and an amount of noise of a preview image based on at least one of spatial resolution, contrast resolution, and an amount of noise of the obtained radiographic image.

In operation S620, the radiographic image photographing apparatus 100 stores the preview image having the changed at least one of the spatial resolution, the contrast resolution, and the amount of noise as an updated preview image.

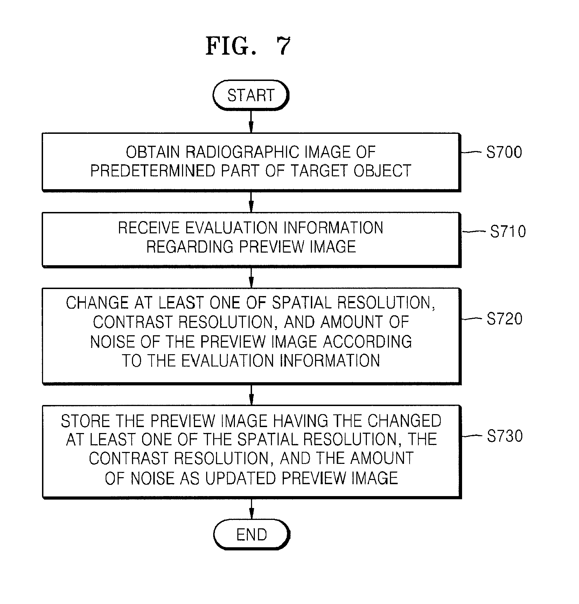

FIG. 7 is a flowchart of a preview image updating method in a radiographic image photographing method, according to another embodiment of the present invention.

In operation S700, the radiographic image photographing apparatus 100 obtains a radiographic image of a predetermined part of a target object according to a parameter value set by a user.

In operation S710, the radiographic image photographing apparatus 100 receives evaluation information regarding a preview image from the user. The user may input the evaluation information by using an input unit such as a keyboard, a voice command, a mouse, and a touch panel. However, it is not limited thereto.

In operation S720, the radiographic image photographing apparatus 100 changes at least one of spatial resolution, contrast resolution, and an amount of noise of the preview image according to the received evaluation information.

In operation S730, the radiographic image photographing apparatus 100 stores the preview image having the changed at least one of the spatial resolution, the contrast resolution, and the amount of noise as an updated preview image.

The methods according to the above-described example embodiments may be recorded in non-transitory computer-readable media including program instructions to implement various operations embodied by a computer. The media may also include, alone or in combination with the program instructions, data files, data structures, and the like. The program instructions recorded on the media may be those specially designed and constructed for the purposes of the example embodiments, or they may be of the kind well-known and available to those having skill in the computer software arts. Examples of non-transitory computer-readable media include magnetic media such as hard disks, floppy disks, and magnetic tape; optical media such as CD ROM disks and DVDs; magneto-optical media such as optical disks; and hardware devices that are specially configured to store and perform program instructions, such as read-only memory (ROM), random access memory (RAM), flash memory, and the like.. Examples of program instructions include both machine code, such as produced by a compiler, and files including higher level code that may be executed by the computer using an interpreter. The described hardware devices may be configured to act as one or more software modules in order to perform the operations of the above-described example embodiments, or vice versa.

The embodiments can be implemented in computing hardware (computing apparatus) and/or software, such as (in a non-limiting example) any computer that can store, retrieve, process and/or output data and/or communicate with other computers. The results produced can be displayed on a display of the computing hardware. A program/software implementing the embodiments may be recorded on non-transitory computer-readable media comprising computer-readable recording media. Examples of the computer-readable recording media include a magnetic recording apparatus, an optical disk, a magneto-optical disk, and/or a semiconductor memory (for example, RAM, ROM, etc.). Examples of the magnetic recording apparatus include a hard disk device (HDD), a flexible disk (FD), and a magnetic tape (MT). Examples of the optical disk include a DVD (Digital Versatile Disc), a DVD-RAM, a CD-ROM (Compact Disc - Read Only Memory), and a CD-R (Recordable)/RW.

While the present invention has been particularly shown and described with reference to exemplary embodiments thereof, it will be understood by those of ordinary skill in the art that various changes in form and details may be made therein without departing from the spirit and scope of the present invention as defined by the following claims.

## Claims

1. A radiographic image photographing method using a radiographic image photographing apparatus, the radiographic image photographing method comprising:
displaying a parameter value of the radiographic image photographing apparatus for a predetermined part of a target object and a preview image corresponding to the parameter value;
changing the parameter value or the preview image based on a user's input; and
changing the preview image according to the changed parameter value, or the parameter value according to the changed preview image.

2. The radiographic image photographing method of claim 1, wherein the displaying of the preview image comprises: displaying the preview image corresponding to the predetermined part of the target object.

3. The radiographic image photographing method of claim 1 or 2, wherein the displaying of the preview image comprises: displaying the preview image corresponding to at least one of a tube voltage, a tube current, a scan type, the number of images, spaces between images, start and end points of each image, a scan field of view (SFOV), a thickness of each image, and a reconfiguration algorithm included in the parameter value.

4. The radiographic image photographing method of claim 3, wherein the changing of the preview image according to the changed parameter value comprises: changing at least one of spatial resolution, contrast resolution, and an amount of noise of the preview image according to a change level of at least one of the tube current and the tube voltage included in the parameter value.

5. The radiographic image photographing method of claim 3 or 4, wherein the changing of the preview image according to the changed parameter value comprises: changing at least one of the tube current and the tube voltage included in the parameter value according to a change level of at least one of spatial resolution, contrast resolution, and an amount of noise of the preview image.

6. The radiographic image photographing method of any of claims 1-5, wherein the displaying comprises: displaying the parameter value on a first region of a display unit of the radiographic image photographing apparatus, and the preview image on a second region thereof.

7. The radiographic image photographing method of any of claims 1-6, wherein the displaying of the preview image comprises: displaying a preview image corresponding to examinee information.

8. The radiographic image photographing method of claim 7, wherein the examinee information comprises at least one of age, weight, height, a body mass index (BMI), and gender of an examinee.

9. The radiographic image photographing method of any of claims 1-8, further comprising:
obtaining a radiographic image of the predetermined part of the target object according to the changed parameter value;
changing at least one of spatial resolution, contrast resolution, and an amount of noise of the preview image based on at least one of spatial resolution, contrast resolution, and an amount of noise of the obtained radiographic image; and
storing the preview image having the changed at least one of the spatial resolution, the contrast resolution, and the amount of noise as an updated preview image.

10. The radiographic image photographing method of any of claims 1-9, further comprising:
obtaining a radiographic image of the predetermined part of the target object according to the changed parameter value;
receiving evaluation information regarding the preview image from a user;
changing at least one of spatial resolution, contrast resolution, and an amount of noise of the preview image according to the received evaluation information; and
storing the preview image having the changed at least one of the spatial resolution, the contrast resolution, and the amount of noise as an updated preview image.

11. The radiographic image photographing method of any of claims 1-10, wherein the preview image comprises a graph image indicating an amount of radiation with respect to the parameter value.

12. An apparatus for photographing a radiographic image of a target object, the apparatus comprising:
a display unit for displaying a parameter value of the radiographic image photographing apparatus for a predetermined part of the target object and a preview image corresponding to the parameter value; and
a control unit for changing the parameter value or the preview image based on a user's input,
wherein the control unit changes the preview image according to the changed parameter value, or the parameter value according to the changed preview image.

13. The apparatus of claim 12, wherein the display unit displays the preview image corresponding to the predetermined part of the target object.

14. The apparatus of claim 12 or 13, wherein the display unit displays the preview image corresponding to at least one of a tube voltage, a tube current, a scan type, the number of images, spaces between images, start and end points of each image, a scan field of view (SFOV), a thickness of each image, and a reconfiguration algorithm included in the parameter value.

15. A computer readable recording medium having recorded thereon a program for executing the radiographic image photographing method of any one of claims 1 through 11.
